# EUROPEAN PATENT APPLICATION

(11) **EP 3 272 670 A1**
(43) Date of publication of application: **24.01.2018**
(21) Application number: 16768520.5
(22) Date of filing: 14.03.2016
(51) Int. Cl.: B65D 83/62, B65D 83/68, A61Q 11/00, A61K 8/73, A61K 8/04, A61K 8/19

(54) **TWO-PACK AEROSOL DENTIFRICE**

(30) Priority: 20.03.2015 JP 2015057824
(71) Applicant: Toyo Aerosol Industry Co., Ltd., Tokyo 141-0022 (JP)
(72) Inventor: NAKAJIMA, Yasutomo, Tokyo 141-0022 (JP); TSUBOUCHI, Makoto, Tokyo 141-0022 (JP); KAMIJYO, Hokuto, Tokyo 141-0022 (JP); NIINOMI, Tomoyuki, Tokyo 141-0022 (JP); IKEDA, Remi, Tokyo 141-0022 (JP)
(74) Representative: Grünecker Patent- und Rechtsanwälte PartG mbB
(86) International application number: PCT/JP2016/057942
(87) International publication number: WO 2016/152617

(57) **Abstract**

The present invention has as its object the provision of a two-liquid mixing-type aerosol toothpaste that has high storage stability and can easily form a high-functional toothpaste composition.

The two-liquid mixing-type aerosol toothpaste of the present invention has a double-structure container including a propellant filling space and two liquid concentrate filling spaces and having a discharging mechanism for simultaneously discharging contents filled in the two liquid concentrate filling spaces. The propellant filling space is filled with a propellant composed of a compressed gas. A first liquid concentrate filling space is filled with a first liquid concentrate composition containing an active ingredient and having a viscosity of 1,000 to 125, 000 mPa·s at a temperature of 20°C. A second liquid concentrate filling space is filled with a second liquid concentrate composition free of the active ingredient and having a viscosity of 1, 000 to 125, 000 mPa·s at a temperature of 20°C.

## Description

### TECHNICAL FIELD

The present invention relates to a two-liquid mixing-type aerosol toothpaste.

### BACKGROUND ART

In the related art, there is known a type of toothpaste that has a tube-type container filled with several types of toothpaste compositions having different constituents (see, for example, Patent Literature 1). These several types of toothpaste compositions can be discharged in the form of stripes from the tube-type container.

To obtain a clear striped pattern of the discharge material, this type of toothpaste requires uniformly squeezing the tube-type container from the bottom without kneading the tube-type container. Since the way to squeeze the tube-type container changes the balance of the amounts of the toothpaste compositions discharged, the several types of toothpaste compositions cannot be squeezed out in a constant quantitative ratio. As a result, there is a problem in that desired effects to be obtained by a combination of several types of toothpaste compositions cannot be obtained stably.

Commonly used toothpaste compositions contain a fluorine compound as an active ingredient. In such a toothpaste composition, the contact between a fluorine compound and calcium carbonate or calcium hydrogen carbonate, which is used as an abrasive or the like, may reduce the effects of the fluorine compound, that is, cavity prevention and enamel remineralization.

### CITATION LIST

### PATENT LITERATURE

Patent Literature 1: Japanese Translation of PCT Patent Application Publication No. Hei. 10-512522

### SUMMARY OF INVENTION

### Technical Problem

The present invention has been made in view of the foregoing circumstances and has as its object the provision of a two-liquid mixing-type aerosol toothpaste of an aerosol dispense. The aerosol toothpaste has high storage stability and can easily form a high-functional toothpaste composition. Solution to Problem

A two-liquid mixing-type aerosol toothpaste of the present invention has a double-structure container including a propellant filling space and two independent liquid concentrate filling spaces and having a discharging mechanism for simultaneously discharging contents filled in the two liquid concentrate filling spaces,
the propellant filling space in the double-structure container is filled with a propellant composed of a compressed gas,
a first liquid concentrate filling space in the double-structure container is filled with a first liquid concentrate composition, and a second liquid concentrate filling space in the double-structure container is filled with a second liquid concentrate composition,
the first liquid concentrate composition is a liquid containing an active ingredient,
the second liquid concentrate composition is a liquid without the active ingredient contained in the first liquid concentrate composition, and
the first liquid concentrate composition and the second liquid concentrate composition each have a viscosity of 1, 000 to 125,000 mPa·s at a temperature of 20°C.

In the two-liquid mixing-type aerosol toothpaste of the present invention, the mixing ratio of the first liquid concentrate composition discharged from the first liquid concentrate filling space to the second liquid concentrate composition discharged from the second liquid concentrate filling space (the mass of the first liquid concentrate composition : the mass of the second liquid concentrate composition) may preferably be from 0.8:1.2 to 1.2:0.8.

In the two-liquid mixing-type aerosol toothpaste of the present invention, the active ingredient may preferably be composed of a fluorine compound.

In the two-liquid mixing-type aerosol toothpaste of the present invention, the second liquid concentrate composition may preferably contain a remineralization promoter.

In the two-liquid mixing-type aerosol toothpaste of the present invention with such a configuration, the remineralization promoter may preferably be composed of calcium phosphate.

### ADVANTAGEOUS EFFECTS OF INVENTION

The two-liquid mixing-type aerosol toothpaste of the present invention has a double-structure container including a discharging mechanism for simultaneously discharging contents filled in two liquid concentrate filling spaces. One of the two liquid concentrate filling spaces is filled with a first liquid concentrate composition, whereas the other is filled with a second liquid concentrate composition. Thus, the first liquid concentrate composition and the second liquid concentrate composition both have a large degree of freedom in formulation design and provide high formulation stability. Even when the first liquid concentrate composition and the second liquid concentrate composition have a high viscosity, the first liquid concentrate composition and the second liquid concentrate composition can always be discharged in a constant quantitative ratio. Moreover, the long-term storage stability can be obtained because neither the first liquid concentrate composition nor the second liquid concentrate composition is exposed to the air outside the container during application.

The two-liquid mixing-type aerosol toothpaste of the present invention has high storage stability and can easily form a high-functional toothpaste composition.

### BRIEF DESCRIPTION OF DRAWINGS

[FIG. 1] is an explanatory view illustrating an example structure of a double-structure container used for a two-liquid mixing-type aerosol toothpaste of the present invention.
[FIG. 2] is a sectional view illustrating a cross section taken along A-A' in FIG. 1.
[FIG. 3] is an explanatory view illustrating another example structure of a double-structure container used for a two-liquid mixing-type aerosol toothpaste of the present invention.

### DESCRIPTION OF EMBODIMENTS

A two-liquid mixing-type aerosol toothpaste of the present invention has a double-structure container including a propellant filling space and two independent liquid concentrate filling spaces and having a discharging mechanism for simultaneously discharging the contents filled in these two liquid concentrate filling spaces. In this double-structure container, the propellant filling space is filled with a propellant composed of a compressed gas. A first liquid concentrate filling space is filled with a first liquid concentrate composition, and a second liquid concentrate filling space is filled with a second liquid concentrate composition.

In the two-liquid mixing-type aerosol toothpaste of the present invention, the first liquid concentrate composition and the second liquid concentrate composition simultaneously discharged from the first liquid concentrate filling space and the second liquid concentrate filling space, respectively, are used in combination to form a high-functional toothpaste composition.

In the two-liquid mixing-type aerosol toothpaste of the present invention, a toothpaste composition formed by combining the first liquid concentrate composition and the second liquid concentrate composition contains basic components, active ingredients, and, as necessary, other optional components. The first liquid concentrate composition and the second liquid concentrate composition have different compositions from each other.

Specifically, in the two-liquid mixing-type aerosol toothpaste of the present invention, the first liquid concentrate composition is a liquid containing an active ingredient, and the second liquid concentrate composition is a liquid without the active ingredient contained in the first liquid concentrate composition. In other words, the second liquid concentrate composition does not contain the active ingredient contained in the first concentrate composition but contains another active ingredient (an active ingredient other than the active ingredient contained in the first liquid concentrate composition) as necessary.

Examples of the basic components in the toothpaste composition includes a cleaning component (polishing component), a foaming component, a moisturizing component (wetting component), a caking component, a flavor and a preservative.

Examples of the cleaning component include calcium carbonate, calcium hydrogenphosphate, silicic acid anhydride, aluminum hydroxide, bentonite and titanium oxide.

Examples of the foaming component include lauroylsarcosine sodium, sodium lauryl sulfate and sucrose fatty acid ester.

Examples of the moisturizing component include sorbitol, glycerol, propylene glycol, polyethylene glycol and 1,3-butylene glycol.

Examples of the caking component include sodium alginate, carboxymethyl cellulose, hydroxyethyl cellulose, xanthan gum, polyvinylpyrrolidone and carrageenan.

Examples of the flavor include saccharin, saccharin sodium and menthol.

Examples of the preservative include sodium benzoate.

Examples of the active ingredients in the toothpaste composition include a fluorine compound, a remineralization promoter, an anti-inflammatory component, an enzyme, a whitening component and an antibacterial component.

Examples of the fluorine compound include sodium fluoride, sodium monofluorophosphate and tin fluoride.

Examples of the remineralization promoter include calcium phosphate.

Examples of the anti-inflammatory component include lysozyme chloride, methyl salicylate and dipotassium glycyrrhizinate.

Examples of the enzyme include dextranase.

Examples of the whitening component (a component for whitening teeth) include urea peroxide, polyethylene glycol, sodium polyphosphate and sodium metaphosphate.

Examples of the antibacterial component include chlorhexidine, triclosan, isopropyl methylphenol and thymol.

Examples of other components in the toothpaste composition include a coloring agent, a solvent (specifically, for example, purified water and ethanol) and a solubilizer (specifically, for example, polyoxyethylene hydrogenated castor oil).

Specific examples of the two-liquid mixing-type aerosol toothpaste of the present invention include a two-liquid mixing-type aerosol toothpaste in which the first liquid concentrate composition contains a fluorine compound as an active ingredient and the second liquid concentrate composition contains a substance (for example, calcium carbonate or calcium hydrogen carbonate) that causes a reaction upon contact with the fluorine compound. In this two-liquid mixing-type aerosol toothpaste, the first liquid concentrate composition does not contain a substance that causes a reaction upon contact with the fluorine compound, and the second liquid concentrate composition may or may not contain an active ingredient other than the fluorine compound. Specific examples of such a two-liquid mixing-type aerosol toothpaste include a two-liquid mixing-type aerosol toothpaste in which the first liquid concentrate composition and the second liquid concentrate composition contain different active ingredients. Specifically, the first liquid concentrate composition contains a fluorine compound as an active ingredient, and the second liquid concentrate composition contains, as an active ingredient, a remineralization promoter composed of calcium phosphate.

### First Liquid Concentrate Composition:

The first liquid concentrate composition may preferably have a viscosity of 1,000 to 125,000 mPa·s, more preferably 5,000 to 50,000 mPa·s at a temperature of 20°C.

If the viscosity of the first liquid concentrate composition is too high, it is difficult to discharge the first liquid concentrate composition. In addition, the first liquid concentrate composition may not be discharged in a desired amount associated with the amount of the second liquid concentrate composition discharged.

If the viscosity of the first liquid concentrate composition is too low, dripping may occur.

### Second Liquid Concentrate Composition:

The second liquid concentrate composition may preferably have a viscosity of 1,000 to 125,000 mPa-s, more preferably 5,000 to 50,000 mPa·s at a temperature of 20°C.

If the viscosity of the second liquid concentrate composition is too high, it is difficult to discharge the second liquid concentrate composition. In addition, the second liquid concentrate composition may not be discharged in a desired amount associated with the amount of the first liquid concentrate composition discharged.

If the viscosity of the second liquid concentrate composition is too low, dripping may occur.

### Propellant:

A compressed gas is used as a propellant.

Examples of the compressed gas include nitrous oxide gas, nitrogen gas, carbon dioxide gas and a mixture of these gases.

This propellant is not discharged from the propellant filling space into the outside of the double-structure container along with simultaneous discharge of the first liquid concentrate composition and the second liquid concentrate composition.

The propellant may preferably be enclosed such that the pressure applied when the double-structure container is filled with the propellant is 0.3 to 1.2 MPa at 25°C.

If the pressure applied when the double-structure container is filled with the propellant (product inner pressure) is too high or too low, in both cases, the contents may not be sprayed in favorable conditions.

### Double-Structure Container:

The double-structure container of the two-liquid mixing-type aerosol toothpaste of the present invention includes a propellant filling space to be filled with a propellant, a first liquid concentrate filling space to be filled with a first liquid concentrate composition, and a second liquid concentrate filling space to be filled with a second liquid concentrate composition. The double-structure container further includes a discharging mechanism for simultaneously discharging the first liquid concentrate composition and the second liquid concentrate composition from the first liquid concentrate filling space and the second liquid concentrate filling space, respectively.

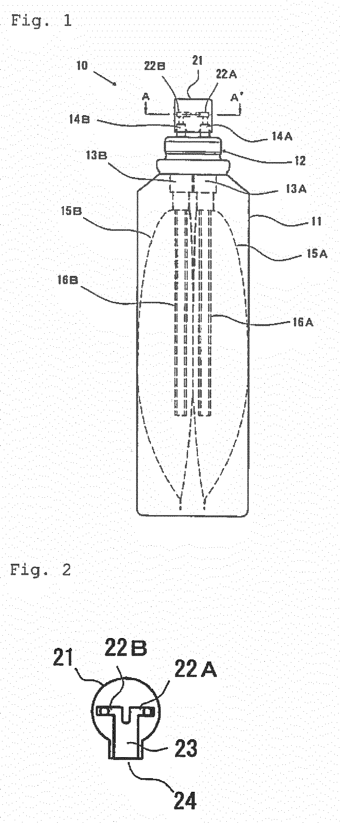

Specific examples of the double-structure container according to the present invention include the following two containers illustrated in FIG. 1 to FIG. 3.

FIG. 1 is an explanatory view illustrating an example structure of the double-structure container used for the two-liquid mixing-type aerosol toothpaste of the present invention. FIG. 2 is a sectional view illustrating a cross section taken along A-A' in FIG. 1.

This double-structure container 10 includes a pressure resistant container 11 made of metal and provided with an aerosol valve 12. The pressure resistant container 11 is provided thereinside with a first inner bag 15A that is formed of, for example, an aluminum laminated film and that defines a first liquid concentrate filling space to be filled with the first liquid concentrate composition, and a second inner bag 15B that is formed of, for example, an aluminum laminated film and that defines a second liquid concentrate filling space to be filled with the second liquid concentrate composition. In the pressure resistant container 11, a propellant filling space to be filled with the propellant is formed from a gap defined by the pressure resistant container 11, the first inner bag 15A, and the second inner bag 15B. The aerosol valve 12 is provided with a first stem 14A and a second stem 14B each having a stem passage inside. The first stem 14A and the second stem 14B are disposed to be movable up and down inside a first housing 13A and a second housing 13B, respectively. A common actuator 21 is disposed on the upper ends of the first stem 14A and the second stem 14B.

In the example illustrated in the figure, a reference character 16A denotes a first dip tube in communication with the stem passage in the first stem 14A at the lower end of the first housing 13A. The first dip tube 16A extends inside the first inner bag 15A toward the bottom of the pressure resistant container 11. A reference character 16B denotes a second dip tube in communication with the stem passage in the second stem 14B at the lower end of the second housing 13B. The second dip tube 16B extends inside the second inner bag 15B toward the bottom of the pressure resistant container 11.

In FIG. 1, the components located inside the pressure resistant container 11 and the actuator 21 are drawn with broken lines.

The common actuator 21 contains a first actuator passage 22A in communication with the stem passage in the first stem 14A, a second actuator passage 22B in communication with the stem passage in the second stem 14B, and a discharge space 23 in communication with, at its end, the first actuator passage 22A and the second actuator passage 22B and forming, at its another end, a discharge port 24.

The actuator 21 common to the first stem 14A for the first inner bag 15A and the second stem 14B for the second inner bag 15B is provided accordingly so as to form the discharging mechanism for simultaneously discharging the first liquid concentrate composition filled in the first inner bag 15A and the second liquid concentrate composition filled in the second inner bag 15B from the first inner bag 15A and the second inner bag 15B, respectively.

In the double-structure container 10 having such a structure, the first inner bag 15A is filled with the first liquid concentrate composition, the second inner bag 15B is filled with the second liquid concentrate composition, and the propellant filling space is filled with a propellant. The inside of the pressure resistant container 11 is always pressurized with the propellant accordingly. Therefore, when the actuator 21 is actuated (depressed), the pressure of the propellant shrinks the first inner bag 15A and the second inner bag 15B, which causes the first liquid concentrate composition and the second liquid concentrate composition to be discharged simultaneously from the first inner bag 15A and the second inner bag 15B, respectively. As a result, the first liquid concentrate composition and the second liquid concentrate composition are discharged from the discharge port 24 of the actuator 21.

Specifically, while the actuator 21 is not actuated or depressed in the double-structure container 10 filled with the first liquid concentrate composition, the second liquid concentrate composition, and the propellant, the first stem 14A and the second stem 14B are being pushed up to block the stem passage in the first stem 14A and the stem passage in the second stem 14B from the inside of the pressure resistant container 11. While the actuator 21 is actuated or depressed, the first stem 14A and the second stem 14B are pushed down, so that the stem passage in the first stem 14A and the stem passage in the second stem 14B simultaneously communicate with the inside of the pressure resistant container 11. The first liquid concentrate composition in the first inner bag 15A and the second liquid concentrate composition in the second inner bag 15B are discharged simultaneously through the fluid passages formed by the first dip tube 16A and the second dip tube 16B, respectively. The first liquid concentrate composition and the second liquid concentrate composition thus simultaneously discharged reach the discharge space 23 through the stem passage in the first stem 14A and the stem passage in the second stem 14B and through the first actuator passage 22A and the second actuator passage 22B, respectively. The first liquid concentrate composition and the second liquid concentrate composition are discharged from the discharge port 24 without being mixed during passage through the discharge space 23. The first liquid concentrate composition and the second liquid concentrate composition discharged from the discharge port 24 are placed on, for example, a toothbrush and mixed at an application site during tooth brushing to form a toothpaste composition.

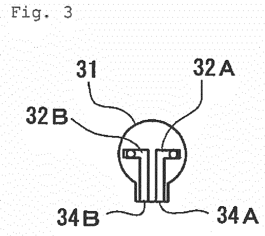

FIG. 3 is an explanatory view illustrating another example structure of a double-structure container used for the two-liquid mixing-type aerosol toothpaste of the present invention. Specifically, FIG. 3 is an explanatory sectional view illustrating the structure of an actuator in accordance with the double-structure container.

This double-structure container has the same structure as that of the double-structure container 10 in accordance with FIG. 1 and FIG. 2 except that the actuator 21 is replaced by an actuator 31 having two discharge ports (specifically, a first discharge port 34A and a second discharge port 34B) and the first liquid concentrate composition and the second liquid concentrate composition are separately discharged from these two discharge ports, respectively.

In other words, the double-structure container in accordance with FIG. 3 includes the actuator 31 and a pressure resistant container having the same structure as that of the pressure resistant container 11 in the double-structure container 10 in accordance with FIG. 1 and FIG. 2.

The actuator 31 is provided with a first actuator passage 32A in communication with, at one end, the stem passage in the first stem and forming a first discharge port 34A at another end, and a second actuator passage 32B in communication with, at one end, the stem passage in the second stem and forming a second discharge port 34B at another end.

The actuator 31 is an actuator common to the first stem and the second stem and is disposed at the upper ends of the first stem and the second stem, like the actuator 21 in the double-structure container 10 in accordance with FIG. 1 and FIG. 2.

While the actuator 31 is actuated or depressed in the double-structure container having such a structure and filled with the first liquid concentrate composition, the second liquid concentrate composition, and the propellant, the first liquid concentrate composition in the first inner bag and the second liquid concentrate composition in the second inner bag are discharged simultaneously. The first liquid concentrate composition is discharged from the first discharge port 34A through the stem passage in the first stem and through the first actuator passage 32A, whereas the second liquid concentrate composition is discharged from the second discharge port 34B through the stem passage in the second stem and through the second actuator passage 32B. The first liquid concentrate composition and the second liquid concentrate composition discharged from the discharge port 24 are placed on, for example, a toothbrush and mixed at an application site during tooth brushing to form a toothpaste composition.

In the double-structure container having the structure described above, the discharging mechanism enables the first liquid concentrate composition filled in the first liquid concentrate filling space and the second liquid concentrate composition filled in the second liquid concentrate filling space to be discharged simultaneously. The discharging mechanism further enables the amount of the first liquid concentrate composition discharged from the first liquid concentrate filling space and the amount of the second liquid concentrate composition discharged from the second liquid concentrate filling space to be controlled at an appropriate quantitative ratio, specifically, so as to be substantially the same.

In the two-liquid mixing-type aerosol toothpaste of the present invention, the mixing ratio of the first liquid concentrate composition discharged from the first liquid concentrate filling space to the second liquid concentrate composition discharged from the second liquid concentrate filling space (the mass of the first liquid concentrate composition : the mass of the second liquid concentrate composition) may preferably be from 0.8:1.2 to 1.2:0.8.

In other words, the amount of the first liquid concentrate composition discharged from the first liquid concentrate filling space and the amount of the second liquid concentrate composition discharged from the second liquid concentrate filling space each may preferably fall within a range of ±20% of the mean of the amounts of the first liquid concentrate composition discharged and the second liquid concentrate composition discharged.

The mixing ratio (the mass of the first liquid concentrate composition : the mass of the second liquid concentrate composition) can be controlled within the above-described range by setting, for example, the viscosity of the first liquid concentrate composition at a temperature of 20°C to 1,000 to 125,000 mPa·s, and the viscosity of the second liquid concentrate composition at a temperature of 20°C to 1,000 to 125,000 mPa·s.

If the mixing ratio (the mass of the first liquid concentrate composition : the mass of the second liquid concentrate composition) is out of the above-described range, the amount of the first liquid concentrate composition discharged from the first liquid concentrate filling space is significantly different from the amount of the second liquid concentrate composition discharged from the second liquid concentrate filling space. Therefore, a mixed discharge material (specifically, the first liquid concentrate composition and the second liquid concentrate composition), i.e., the toothpaste composition, may fail to have desired functions.

The two-liquid mixing-type aerosol toothpaste of the present invention described above is produced by filling the first liquid concentrate filling space and the second liquid concentrate filling space in the double-structure container with the first liquid concentrate composition and the second liquid concentrate composition, respectively, and filling the propellant filling space with the propellant.

The two-liquid mixing-type aerosol toothpaste of the present invention has a double-structure container including a discharging mechanism for simultaneously discharging contents filled in two liquid concentrate filling spaces. One of the two liquid concentrate filling spaces is filled with a first liquid concentrate composition, whereas the other is filled with a second liquid concentrate composition.

Thus, the first liquid concentrate composition and the second liquid concentrate composition both have a large degree of freedom in formulation design and provide high formulation stability.

Since the first liquid concentrate composition and the second liquid concentrate composition can be simultaneously discharged in suitable amounts (specifically, in the same amount) from the two liquid concentrate filling spaces in the double-structure container, respectively, the first liquid concentrate composition and the second liquid concentrate composition, even if having high viscosity, can always be discharged in a constant quantitative ratio. As a result, there is no possibility that the amount of one liquid concentrate composition discharged is much larger than the amount of another liquid concentrate composition discharged. As a result, a toothpaste composition formed by combining the first liquid concentrate composition and the second liquid concentrate composition and having desired functions can always be obtained by simply operating the discharging mechanism, specifically, for example, only depressing the actuator once (one push) to discharge the first liquid concentrate composition and the second liquid concentrate composition.

Moreover, the long-term storage stability can be obtained because neither the first liquid concentrate composition nor the second liquid concentrate composition is exposed to the air outside the container during application.

Therefore, the two-liquid mixing-type aerosol toothpaste of the present invention can have high storage stability and can easily form a high-functional toothpaste composition.

In the two-liquid mixing-type aerosol toothpaste of the present invention, compositions that are considered difficult to use in combination in the same stock solution from the viewpoint of the formulation stability of active ingredients can be stored in combination in the same container without any undesirable effects and can be used together. Specifically, for example, a liquid concentrate composition containing a fluorine compound and a liquid concentrate composition containing calcium carbonate or calcium hydrogen carbonate, which may reduce the effects of the fluorine compound (specifically, cavity prevention and enamel remineralization) upon contact with the fluorine compound, can be filled in a single double-structure container for storage. For example, a liquid concentrate composition containing a fluorine compound and a liquid concentrate composition containing calcium phosphate can be filled in a single double-structure container for storage.

In the two-liquid mixing-type aerosol toothpaste of the present invention, a non-flammable compressed gas is used as a propellant for the first liquid concentrate composition and the second liquid concentrate composition. The use of the non-flammable compressed gas provides high safety irrespective of the operation environment and eliminates the risk of an explosion accident in discarding the double-structure container.

### EXAMPLES:

Examples of the present invention will be described below, but the present invention is not limited by these.

### [Example 1]

### Preparation of First Liquid Concentrate Composition:

A first liquid concentrate composition was prepared by mixing the components described below.

The viscosity of the obtained first liquid concentrate composition at a temperature of 20°C was measured using a BM-type rotary viscometer (rotor No. 3, 12 rpm, after 1 minute) and found to be 6,000 mPa·s.

### Constituents of First Liquid Concentrate Composition:

Fluorine compound: 0.10% by mass
Coloring agent: 0.01% by mass
Water: 48.39% by mass
Sorbitol: 25.00% by mass
Glycerol: 25.00% by mass
Carboxymethyl cellulose: 1.00% by mass
Flavor: 0.10% by mass
Saccharin: 0.20% by mass
Sodium benzoate: 0.20% by mass
Total: 100.0% by mass

### Preparation of Second Liquid Concentrate Composition:

A second liquid concentrate composition was prepared by mixing the components described below.

The viscosity of the obtained second liquid concentrate composition at a temperature of 20°C was measured using a BM-type rotary viscometer (rotor No. 3, 12 rpm, after 1 minute) and found to be 8,000 mPa·s.

### Constituents of Second Liquid Concentrate Composition:

Calcium phosphate: 1.00% by mass
Water: 37.49% by mass
Sorbitol: 25.00% by mass
Glycerol: 25.00% by mass
Carboxymethyl cellulose: 1.00% by mass
Calcium carbonate: 10.00% by mass
Flavor: 0.10% by mass
Saccharin: 0.20% by mass
Sodium benzoate: 0.20% by mass
Coloring agent: 0.01% by mass
Total: 100.0% by mass

### Production of Aerosol Product:

A two-liquid mixing-type aerosol toothpaste was produced by: preparing a double-structure container having the structure illustrated in FIG. 1 and FIG. 2; filling a first liquid concentrate filling space (first inner bag) in the double-structure container with the first liquid concentrate composition; filling a second liquid concentrate filling space (second inner bag) with the second liquid concentrate composition; and filling a propellant filling space with nitrogen gas as a propellant such that the product inner pressure in the double-structure container was 0.7 MPa at 25°C.

The two-liquid mixing-type aerosol toothpaste in Example 1 was applied just after production. As a result, it was found that this aerosol toothpaste exhibited superior functions attributed to the first liquid concentrate composition and the second liquid concentrate composition.

It was also found that, even after storage in an environment at a temperature of 45°C for a long period of time or for one month, the two-liquid mixing-type aerosol toothpaste in Example 1 provided superior functions and comfort during use that were similar to those obtained just after production.

### Reference Signs List

- 10: double-structure container
- 11: pressure resistant container
- 12: aerosol valve
- 13A: first housing
- 13B: second housing
- 14A: first stem
- 14B: second stem
- 15A: first inner bag
- 15B: second inner bag
- 16A: first dip tube
- 16B: second dip tube
- 21: actuator
- 22A: first actuator passage
- 22B: second actuator passage
- 23: discharge space
- 24: discharge port
- 31: actuator
- 32A: first actuator passage
- 32B: second actuator passage
- 34A: first discharge port
- 34B: second discharge port

## Claims

1. A two-liquid mixing-type aerosol toothpaste comprising:
a double-structure container including a propellant filling space and two independent liquid concentrate filling spaces and having a discharging mechanism for simultaneously discharging contents filled in the two liquid concentrate filling spaces, wherein
the propellant filling space in the double-structure container is filled with a propellant composed of a compressed gas,
a first liquid concentrate filling space in the double-structure container is filled with a first liquid concentrate composition, and a second liquid concentrate filling space in the double-structure container is filled with a second liquid concentrate composition,
the first liquid concentrate composition is a liquid containing an active ingredient,
the second liquid concentrate composition is a liquid without the active ingredient contained in the first liquid concentrate composition, and
the first liquid concentrate composition and the second liquid concentrate composition each have a viscosity of 1, 000 to 125,000 mPa·s at a temperature of 20°C.

2. The two-liquid mixing-type aerosol toothpaste according to claim 1, wherein a mixing ratio of the first liquid concentrate composition discharged from the first liquid concentrate filling space to the second liquid concentrate composition discharged from the second liquid concentrate filling space (a mass of the first liquid concentrate composition : a mass of the second liquid concentrate composition) is from 0.8:1.2 to 1.2:0.8.

3. The two-liquid mixing-type aerosol toothpaste according to claim 1 or 2, wherein the active ingredient is composed of a fluorine compound.

4. The two-liquid mixing-type aerosol toothpaste according to any one of claims 1 to 3, wherein the second liquid concentrate composition contains a remineralization promoter.

5. The two-liquid mixing-type aerosol toothpaste according to claim 4, wherein the remineralization promoter is composed of calcium phosphate.
